# EUROPÄISCHE PATENTANMELDUNG

(11) **EP 2 060 278 A1**
(43) Veröffentlichungstag der Anmeldung: **20.05.2009**
(21) Anmeldenummer: 08450182.4
(22) Anmeldetag: 13.11.2008
(51) Int. Cl.: A61L 2/22, A61L 2/24, A61L 9/14, A61L 9/015

(54) **Verfahren zur Dekontamination eines Raumes sowie Vorrichtung hierfür**

(30) Priorität: 13.11.2007 AT 18282007
(71) Anmelder: ORTNER REINRAUMTECHNIK GMBH, 9500 Villach (AT)
(72) Erfinder: Ortner, Josef, 9500 Villach (AT)
(74) Vertreter: Sonn & Partner Patentanwälte

(57) **Zusammenfassung**

Die Erfindung betrifft ein Verfahren zur Dekontamination eines Raums (1) mit einem Reinigungsgas/Luft-Gemisch, wobei das Reinigungsgas/Luft-Gemisch über einen vorbestimmten Zeitraum im Wesentlichen unidirektional in den Raum (1) eingebracht wird, so dass sich ein Freistrahl ausbildet, wobei die unidirektionale Strömungsrichtung beibehalten wird, bis sich eine stationäre Strömung ausgebildet hat, und anschließend die unidirektionale Strömungsrichtung zur Reinigung des gesamten Raums (1) geändert wird. Weiters betrifft die Erfindung eine Vorrichtung (2) zur Dekontamination, bei der die zumindest eine Düse (12) während der Einbringung des Reinigungsgas/Luft-Gemisches stationär angeordnet ist, so dass Reinigungsgas/Luft-Gemisch für einen vorbestimmten Zeitraum im Wesentlichen unidirektional über die Düse (12) in den Raum (1) einleitbar ist, wobei das Reinigungsgas/Luft-Gemisch über die zumindest eine Düse (12) zumindest in einer zweiten, unterschiedlichen Richtung unidirektional einleitbar ist.

## Beschreibung

Die Erfindung betrifft ein Verfahren zur Dekontamination eines Raums mit einem Reinigungsgas/Luft-Gemisch sowie eine Vorrichtung zur Dekontamination eines Raums mit einem Reinigungsgas/Luft-Gemisch mit zumindest einer Düse, über welche das Reinigungsgas/Luft-Gemisch in den Raum eingebracht wird.

In der pharmazeutischen und chemischen Industrie, ebenso wie in medizinischen und kosmetischen Einrichtungen und in Labor- und Versuchsanstalten müssen geschlossene Räume, d.h. diverse Kammern und größere Räume, samt gegebenenfalls vorhandenem Inventar in gewissen Abständen dekontaminiert werden. Hierfür wird üblicherweise ein Reinigungsgas, insbesondere Wasserstoffperoxid, verwendet, das so lange in den Raum eingebracht wird, bis die gewünschte Konzentration in der Raumluft erreicht ist. Für die Einbringung des Reinigungsgases sind verschiedenste Verfahren bzw. Vorrichtungen bekannt, wobei üblicherweise das Reinigungsgas zunächst in gesättige Form gebracht wird (dies wird bei Wasserstoffperoxid mittels Generatoren bewerkstelligt) und das Reiningungsgas wird sodann in den zu dekontaminierenden Raum eingebracht. Hierbei gilt es den Begasungszyklus möglichst kurz zu halten, wobei in kürzester Zeit nach Möglichkeit der ganze Raum, insbesondere auch Nischen und Winkel, mit dem Reiningungsgas erreicht werden soll(en). Hierfür werden bisher im Raum freistehende Ventilatoren eingesetzt oder jedoch insbesondere auch Düsen, die um zwei senkrecht zueinander angeordnete Achsen rotieren.

So ist beispielsweise aus der WO02/16045 A eine Düse zur Dekontamination von Räumen mittels Wasserstoffperoxid bekannt. Eine derartige Düse kann im Deckenbereich des zu reinigenden Raums angeordnet sein, wobei eine Rotation der Düse sowohl um eine senkrecht angeordnete als auch um eine horizontal angeordnete Achse, vorgesehen ist.

Die WO 2003/082355 beschreibt eine kompakte, mobile Vorrichtung zur Dekontamination von geschlossenen Räumen. Die Verteilung des Reinigungsgas/Luft-Gemischs im zu dekontaminierenden Raum wird über eine rotierende Düse erzielt, wobei die Düsenvorrichtung einen Verteilerarm mit zumindest einer Spitze aufweist, dem ein Antrieb zwecks Rotation zugeordnet ist.

Tests haben jedoch gezeigt, dass überraschenderweise durch deratig in allen Raumrichtungen rotierende Düsen keine optimale Verteilung des Reiningungsgases in dem zu reinigenden Raum erzielt wird, sondern derartige Düsen zumeist nur eine Ausbreitung des Reiningungsgases in nächster Umgebung der Düse bewirken.

Ziel der vorliegenden Erfindung ist es demzufolge, die Einbringung des Reiningungsgases derart zu verbessern, dass Räume unterschiedlicher Abmessungen und Ausstattungen möglichst optimal, d.h. möglichst vollständig und in relativ kurzer Zeit, mit dem Reiningungsgas dekontaminiert werden. Zudem soll auch die Spülung des Raums, d.h. die Entfernung des Reiningungsgases aus dem Raum, verbessert werden.

Dies wird bei dem Verfahren der angeführten Art dadurch erzielt, dass das Reinigungsgas/Luft-Gemisch über einen vorbestimmten Zeitraum im Wesentlichen unidirektional in den Raum eingebracht wird, so dass sich ein Freistrahl ausbildet, wobei die unidirektionale Strömungsrichtung beibehalten wird, bis sich eine stationäre Strömung ausgebildet hat, und anschließend die unidirektionale Strömungsrichtung zur Reinigung des gesamten Raums geändert wird.

Durch die unidirektionale Ausrichtung der Reiningungsgas/LuftGemisch-Einbringung anstelle bisher bekannter kontinuierlich rotierender Düsen kann sich ein Freistrahl ausbilden, d.h. der Strahl hat eine Kegelform, wobei die Geschwindigkeiten der Kegelachse am größten ist und radial nach einer Gauss-Verteilung abnimmt. Hierdurch kann auch eine bis an die Wände bzw. Ecken des Raums ausreichende Strömungsgeschwindigkeit und somit eine Durchmischung sichergestellt werden, wobei die gleiche unidirektionale Strömungsausrichtung beibehalten wird, bis sich eine großräumige, stationäre Strömung ausgebildet hat, wodurch eine möglichst große Durchschnittsgeschwindigkeit im gesamten Raum erreicht wird. Die unidirektionale Strömungsrichtung in unterschiedliche Raumrichtungen kann beispielsweise über eine in einem gewissen Intervall veränderte Ausrichtung einer einzigen Düse oder jedoch durch mehrere in unterschiedliche Raumrichtungen ausgerichtete Düsen, über welche zeitlich versetzt zueinander das Reiningungsgas/Luft-Gemisch in den Raum eingeleitet wird, erzielt werden. Computersimulationen des Gasstroms und der Gasverteilung haben gezeigt, dass hierdurch das Reiningungsgas/Luft-Gemisch in eine definierte Walzenform gebracht werden kann, so dass die Eindringtiefe in den Raum sichergestellt ist. Somit ergibt sich ein den gesamten Raum in Form einer Walze erfassender Strom, so dass gegenüber vorbekannten während der Gaseinbringung rotierenden Düsen eine Dekontamination binnen kürzerer Zeit und zudem eine zuverlässige Dekontamination auch in Nischen und Winkeln des Raums erzielt werden kann.

Um den Raum nach der Dekontamination von dem Reinigungsgas zu befreien, ist eine so genannte "Spülung" erforderlich. Auch hierbei hat sich gezeigt, dass die Zeiten für die Spülung des Raums wesentlich reduziert werden können, wenn nach einer vorbestimmten Einwirkzeit des Reinigungsgas/Luft-Gemisches reine Luft in den Raum eingeleitet wird, wobei die Luft über einen vorbestimmten Zeitraum im Wesentlichen unidirektional in den Raum eingeleitet wird, so dass sich ein Freistrahl ausbildet, wobei die unidirektionale Strömungsrichtung beibehalten wird, bis sich eine stationäre Strömung ausgebildet hat, und anschließend die unidirektionale Strömungsrichtung zur Spülung des gesamten Raums geändert wird. Hierdurch kann der Spülzyklus gegenüber bekannten Spülverfahren um 50% bis 70% verkürzt werden.

Um eine Kondensation der in der Raumluft enthaltenen Luftfeuchtigkeit zu vermeiden, ist es von Vorteil, wenn der zu reinigende Raum vor Einbringung des Reinigungsgas/Luft-Gemisches auf eine Luftfeuchtigkeit von ca. 30-40% relative Luftfeuchtigkeit getrocknet wird.

Wenn das Reinigungsgas mit voller Konzentration (z.B. ca. 1000-1500 ppm) in den Raum eingebracht wird - wie dies bei bekannten Verfahren häufig der Fall ist - ist das Risiko relativ hoch, dass im Umfeld der Gaseinbringung in einzelnen Zonen das hoch konzentrierte Gasgemisch direkt auf Oberflächen trifft und somit Beschädigungen verursacht. Demzufolge ist es vorteilhaft, wenn die Konzentration des Reinigungsgases im Reinigungsgas/Luft-Gemisch vor Einbringung in den Raum bestimmt wird. Hierbei kann die zugeführte Luftmenge variabel zwischen 100 m³/h und 300 m³/h gewählt werden, so dass die Konzentration des Reinigungsgas/Luft-Gemisches in dem zu dekontaminierenden Raum wesentlich genauer eingestellt bzw. geregelt werden kann als bisher.

Als günstig hat sich hierbei erwiesen, wenn die Konzentration des Reinigungsgases zwischen 400 und 900 ppm beträgt.

Zur Dekontaminierung von Räumen in den eingangs genannten Industriezweigen hat sich insbesondere als vorteilhaft herausgestellt, wenn als Reinigungsgas Wasserstoffperoxid, Ammoniak, Formaldehyd, Chlor, Ethylenoxid bzw. eine Mischung hievon verwendet wird.

Um eine möglichst effiziente Dekontaminierung des Raums zu gewährleisten, ist es vorteilhaft, wenn die unidirektionalen Strömungsrichtungen, die Gesamtdauer der Begasung sowie die Dauer der Intervalle der Begasung in jeweils eine bestimmte Strömungsrichtung, in Abhängigkeit von verschiedenen Parametern des Raums, insbesondere Größe, Raumform und dergl., mittels Simulation per Computer vorbestimmt wird.

Weiters haben Tests gezeigt, dass sich die gewünschte großräumige, walzenförmige Strömung des Reinigungsgas/Luft-Gemisches insbesondere einstellt, wenn das Reinigungsgas/Luft-Gemisch mit einer Strömungsgeschwindigkeit zwischen 10 und 30 m/s, insbesondere mit ca. 17,5 m/s, eingeleitet wird.

Die Vorrichtung der eingangs angeführten Art ist dadurch gekennzeichnet, dass die zumindest eine Düse während der Einbringung des Reinigungsgas/Luft-Gemisches stationär angeordnet ist, so dass Reinigungsgas/Luft-Gemisch für einen vorbestimmten Zeitraum im Wesentlichen unidirektional über die Düse in den Raum einleitbar ist, wobei das Reinigungsgas/Luft-Gemisch über die zumindest eine Düse zumindest in einer zweiten, unterschiedlichen Richtung unidirektional einleitbar ist. Durch das Vorsehen einer während der Einbringung stationär angeordneten Düse kann sich ebenso wie bei dem vorhergehend beschriebenen Verfahren ein Freistrahl ausbilden, so dass sich nach einem vorbestimmten Zeitraum eine großräumige stationäre Strömung in Form einer Walze in dem Raum ausbildet, so dass gegenüber bekannten rotierenden Düsen eine effizientere und zuverlässigere Dekontamination des Raums erzielt wird. Die unidirektionale Einleitung des Reinigungsgas/Luft-Gemisches kann hierbei über eine einzige Düse erzielt werden, deren Ausrichtung nach dem vorbestimmten Zeitraum intervallmäßig verändert wird.

Vorteilhaft ist es jedoch insbesondere, wenn zumindest zwei in unterschiedliche Richtungen ausgerichtete Düsen vorgesehen sind, wobei jeder Düse ein Verschlussventil zugeordnet ist, so dass für einen vorbestimmten Zeitraum im Wesentlichen unidirektional über eine einzige Düse das Reinigungsgas/Luft-Gemisch in den Raum einleitbar ist. Durch das Vorsehen von zumindest zwei Düsen, die in unterschiedliche Richtungen ausgerichtet sind, ist eine Verstellung der Düsen nach einem vorbestimmten Zeitraum nicht erforderlich, sondern es kann durch einfaches Öffnen und Schließen des jeweiligen Verschlussventils jeweils in die gewünschte Richtung das Reinigungsgas/Luft-Gemisch in den Raum eingebracht werden.

Hinsichtlich einer konstruktiv einfachen Ausgestaltung ist es hierbei günstig, wenn die Düsen an einem, vorzugsweise zylindrischen, gemeinsamen Grundkörper befestigt sind.

Um die Düsen auf einfache Weise mit dem Reinigungsgas bzw. dem Gemisch anspeisen zu können, ist es vorteilhaft, wenn der Grundkörper einen Anschluss zur Einspeisung des Reinigungsgases und/oder des Reinigungsgas/Luft-Gemisches aufweist.

Wenn mit dem Grundkörper eine vorzugsweise kreisringförmige Befestigungsplatte verbunden ist, kann der Grundkörper auf einfache Weise insbesondere an einer Zwischendecke des zu reinigenden Raums befestigt werden. Selbstverständlich kann der Grundkörper jedoch auch an einer mobilen Vorrichtung, z.B. an einem mit Rädern versehenen Befestigungselement angebracht sein, wobei das Befestigungselement vorzugsweise eine höhenverstellbare Hubsäule aufweist.

Um eine möglichst gleichmäßige, effiziente Begasung des gesamten Raums zu erzielen, ist es vorteilhaft, wenn zumindest drei, insbesondere vier, fünf oder sechs Düsen vorgesehen sind. Ebenso ist es für eine gleichmäßige Begasung des gesamten Raums von Vorteil, wenn die Düsen entlang einer Kreisbahn in regelmäßigen Abständen angeordnet sind.

Zur unidirektionalen Einleitung des Reinigungsgas/Luft-Gemisches ist es von Vorteil, wenn die Düsen einen sich konisch verjüngenden Endabschnitt aufweisen, wobei bei der Auslegung der Düse die maßgebliche strömungstechnische Größe der Impulsstrom am Austritt (= Produkt aus Austrittsgeschwindigkeit und Massenstrom) ist. Demzufolge ist ein kleiner Durchmesser mit hoher Austrittsgeschwindigkeit in der Wirkung äquivalent zu einem großen Durchmesser mit kleiner Austrittsgeschwindigkeit.

Es hat sich gezeigt, dass eine effiziente Begasung des gesamten Raums erzielt wird, sofern die Vorrichtung im Deckenbereich befestigt ist, wenn die Längsachse des Endabschnitts einen Winkel zwischen 10° und 20°, insbesondere von im Wesentlichen 15°, zur Horizontalen einschließt.

Um eine unterschiedliche Ausrichtung der Düse zu erhalten und zugleich die Turbulenzen in der Düse möglichst gering zu halten, ist es günstig, wenn ein an den Grundkörper anschließender Abschnitt der Düsen im Wesentlichen kreisbogenförmig gekrümmt ist.

Hinsichtlich einer konstruktiv einfachen Ausgestaltung zum Öffnen bzw. Schließen der jeweiligen Düse ist es vorteilhaft, wenn die Ventile jeweils einen in einer Schließstellung eine Eintrittsöffnung der jeweiligen Düse verschließenden Stößel aufweisen. Hierbei kann zuverlässig ein Öffnen bzw. Schließen der jeweiligen Düse erzielt werden, wenn jedem Stößel eine hydraulische, pneumatische oder elektrische Antriebsvorrichtung zugeordnet ist.

Hinsichtlich einer automatischen Ansteuerung bzw. Regelung, über welche Düse gerade unidirektional das Reinigungsgas/Luft-Gemisch in den Raum eingebracht wird, ist es von Vorteil, wenn zur Ansteuerung der Antriebsvorrichtungen eine Steuer- bzw. Regeleinheit vorgesehen ist.

Um unabhängig von der Einbausituation bzw. der Infrastruktur die Vorrichtung einsetzen zu können, ist es vorteilhaft, wenn in dem Grundkörper ein Ventilator aufgenommen ist. Über den Ventilator kann somit Umluft eingesaugt werden, die in der Vorrichtung selbst mit dem Reinigungsgas zur Erzielung der gewünschten Konzentration vermischt wird, und sodann das Reinigungsgas/Luft-Gemisch über die Düsen in den zu kontaminierenden Raum eingebracht wird.

Alternativ ist es jedoch auch denkbar, dass das Reinigungsgas mit der Luft nicht in der Vorrichtung, insbesondere dem Grundkörper selbst, vermischt wird, sondern zur Mischung des Reinigungsgases und der Luft ein von dem Grundkörper getrennt angeordneter Behälter vorgesehen ist.

Um einen zuverlässigen Betrieb der gesamten Vorrichtung zu gewährleisten, ist es von Vorteil, wenn die gesamte Vorrichtung aus Metall, insbesondere Edelstahl, oder einem Wasserstoffperoxid-beständigen Kunststoff, insbesondere PVC, PC, PTFE oder PE, besteht.

Die Erfindung wird nachfolgend anhand von in den Zeichnungen dargestellten bevorzugten Ausführungsbeispielen, auf die sie jedoch nicht beschränkt sein soll, noch näher erläutert. Im Einzelnen zeigen in den Zeichnungen:
Fig. 1 eine schematische Ansicht einer Begasung eines Raums über die Umluft im Raum, wobei das Reinigungsgas in die Düse eingeleitet wird;
Fig. 2 ein schematisches Blockschaltbild ähnlich Fig. 1, wobei das Reinigungsgas gesondert von der Düse in den Raum eingebracht wird;
Fig. 3 ein schematisches Blockschaltbild ähnlich den Fig. 1 und 2, wobei das Reinigungsgas/Luft-Gemisch in einer externen Einheit hergestellt wird;
Fig. 4 schematisch ein Blockschaltbild ähnlich Fig. 3 mit einer Vielzahl zu dekontaminierenden Räumen;
Fig. 5 eine perspektivische Ansicht einer Vorrichtung zur Dekontamination von Räumen mit sechs unterschiedlich ausgerichteten Düsen;
Fig. 6 eine Ansicht von unten auf die Vorrichtung gemäß Fig. 5;
Fig. 7 schematisch einen Querschnitt der Vorrichtung gemäß den Fig. 5 und 6 mit einem in einem Grundkörper aufgenommenen Ventilator;
Fig. 8 eine Ansicht ähnlich Fig. 7, jedoch ohne Ventilator;
Fig. 9 eine Ansicht von unten auf ein weiteres Ausführungsbeispiel der Vorrichtung mit lediglich vier Düsen;
Fig. 10 eine Ansicht von Teilen der Vorrichtung gemäß Fig. 9;
Fig. 11 eine Draufsicht der Vorrichtung gemäß den Fig. 9 und 10; und
Fig. 12 eine auseinandergezogene, perspektivische Ansicht der Vorrichtung gemäß den Fig. 9 bis 11.

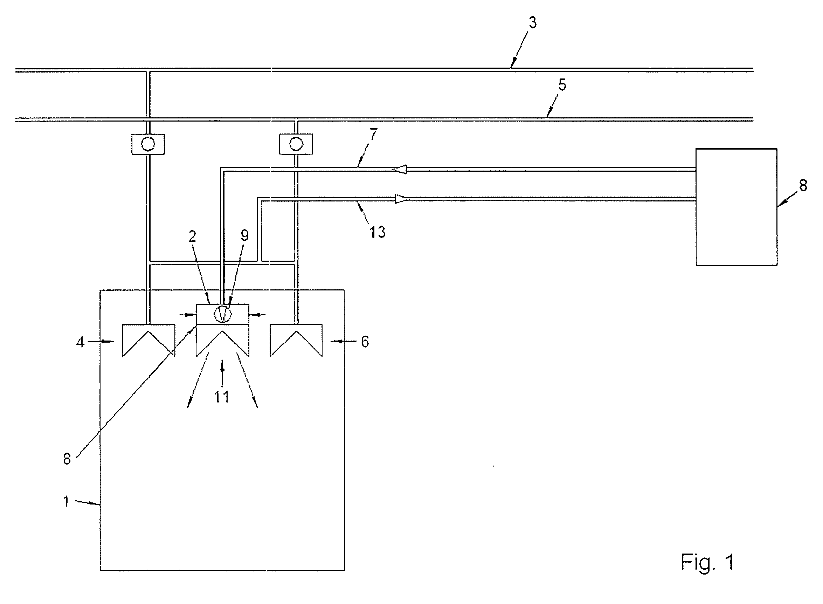

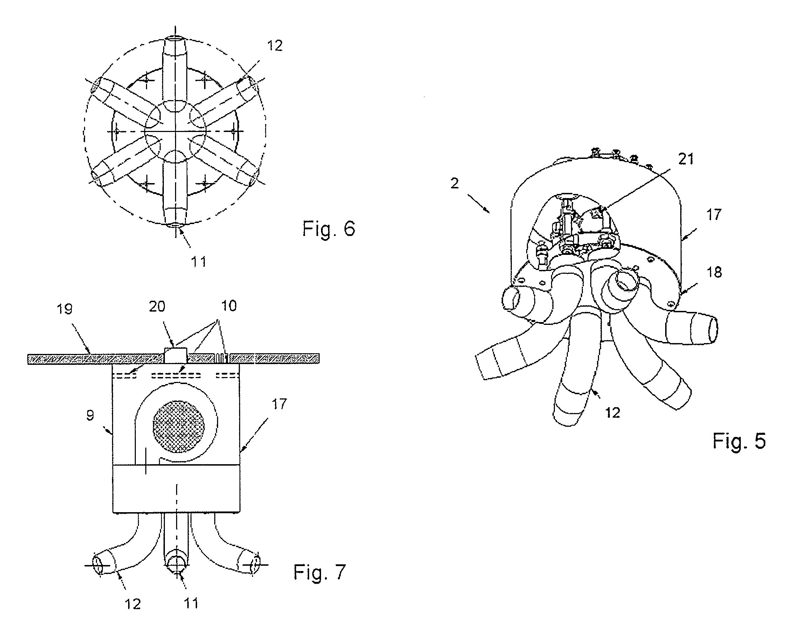

In Fig. 1 ist ein zu dekontaminierender Raum 1 ersichtlich, bei dem im Deckenbereich eine Vorrichtung 2 zur Begasung des Raums mit einem Reinigungsgas/Luft-Gemisch vorgesehen ist. Weiters weist der Raum 1 eine Zuluftleitung 3 mit einer Zuluftöffnung 4 sowie eine Abluftleitung 5 mit einer Abluftöffnung 6 auf. Um in dem Raum 1 eine großräumige, stationäre Strömung in Form einer Walze zu erzielen, wird über eine Reinigungsgas-Zulaufleitung 7 von einem Generator 8, in welchem ein gesättigtes Wasserstoffperoxidgas hergestellt wird, mit einer Konzentration von ca. 1500 ppm in die Vorrichtung 2 gespeist. Die Vorrichtung 2 weist einen Grundkörper 17 auf (vgl. Fig. 7), in welchem ein Hochdruckventilator 9 aufgenommen ist, so dass in dem Grundkörper 17 das über die Reinigungsgas-Zuluftleitung 7 eingespeiste Reinigungsgas (ca. 40 m³/h) mit der aus dem Raum 1 über Ansaugöffnungen 10 (vgl. Fig. 7) eingesaugten Umluft vermischt wird. Somit kann über zumindest eine Düsenöffnung 11 einer Düse 12 (vgl. Fig. 7) das Reinigungsgas/Luft-Gemisch mit einem Durchfluss von ca. 100-300 m³/h in den Raum 1 eingeleitet werden.

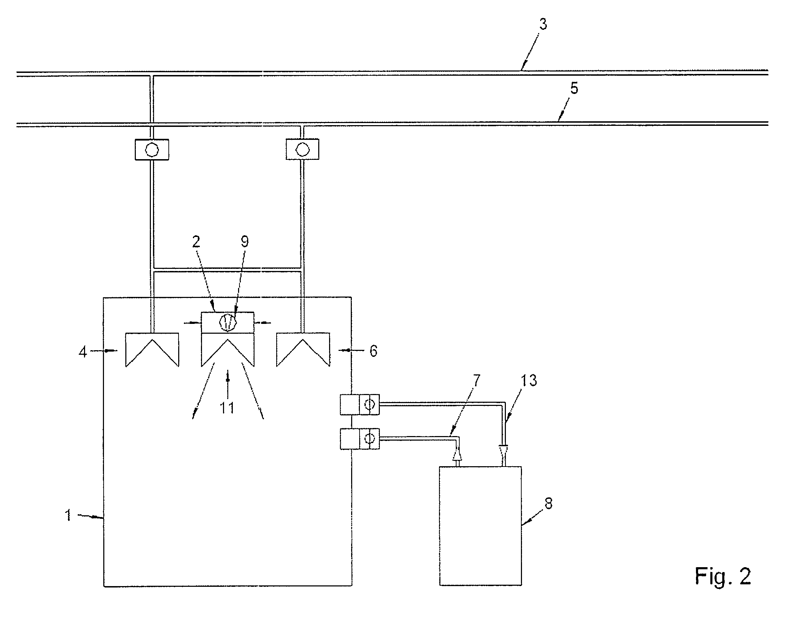

In Fig. 2 ist ein weiteres Ausführungsbeispiel der Erfindung gezeigt, wobei hier ebenfalls im Deckenbereich eine Begasungsvorrichtung 2 vorgesehen ist, die jedoch nicht mit einer Reinigungsgas-Zulaufleitung verbunden ist (dies ist aufgrund baulicher Gegebenheiten häufig nicht möglich). Somit wird das vom Generator 8 erzeugte Wasserstoffperoxid über die Zulaufleitung 7 mit hoher Konzentration in den Raum 1 eingebracht, wobei die Umluft im Raum 1 sodann wieder über den Ventilator 9 der Vorrichtung 2 angesaugt wird und über die Düsenöffnung 11 derart in den Raum eingebracht wird, dass sich eine großräumig stationäre Strömung in Form einer Walze ergibt. Auch hierdurch kann eine effiziente Dekontamination des Raums 1 erzielt werden. Der Generator weist (ebenso wie beim Ausführungsbeispiel gemäß Fig. 1) zudem eine Rücklaufleitung 13 auf, über welche Wasserstoffperoxid mit einer Konzentration von ca. 0-800 ppm in den Generator rückgeleitet werden kann.

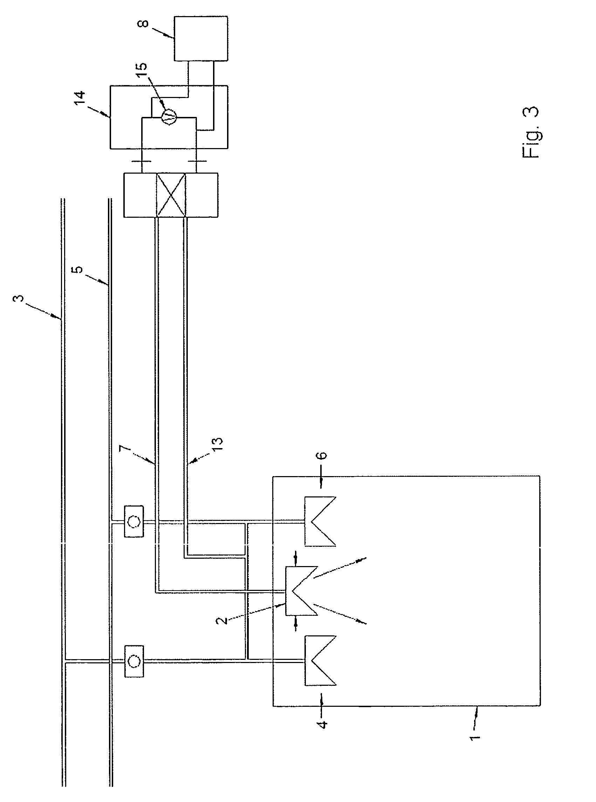

In Fig. 3 ist ein weiteres Ausführungsbeispiel der Erfindung gezeigt, wobei hier die Vorrichtung 2 nicht selbst einen Hochdruckgenerator 9 aufweist, sondern getrennt von der Begasungsvorrichtung 2 eine ISU (Interactive Superinduce Unit)-Einheit 14 vorgesehen ist. An die ISU-Einheit 14 ist der Generator 8 angeschlossen, so dass in der ISU-Einheit 14 mit der Umgebungsluft ein Reinigungsgas/Luft-Gemisch mit einer Konzentration von ca. 0-800 ppm H₂O₂ erzeugt werden kann. Über eine Pumpe 15 wird das Reinigungsgas/Luft-Gemisch sodann über die Zulaufleitung 7 zu der Begasungsvorrichtung 2 geleitet. Auch hier weist der Raum 1 eine Zuluftleitung 3 mit einer Zuluftöffnung 4 und eine Abluftleitung 5 mit einer Abluftöffnung 6 auf. Wesentlich ist jedoch lediglich, dass sich aufgrund der unidirektionalen Einbringung des Reinigungsgas/Luft-Gemisches über die Begasungsvorrichtung 2 eine großräumige stationäre Strömung in Form einer Walze im Raum 1 einstellt, über welche der gesamte Raum 1 auf einfache und effiziente Weise begast werden kann.

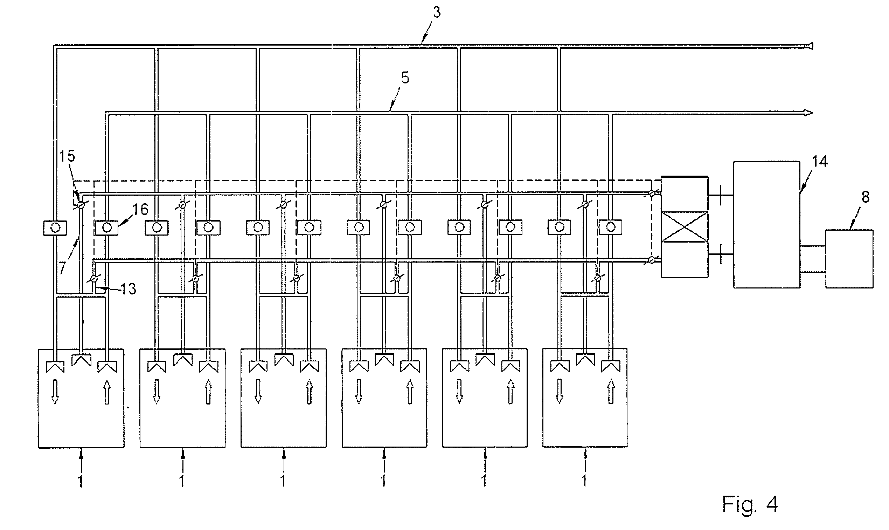

In Fig. 4 ist zudem ersichtlich, dass über eine einzige ISU-Einheit 14 eine Vielzahl unterschiedlicher Räume 1 mit dem Reinigungsgas/Luft-Gemisch begast werden kann. Hierzu weisen die jeweiligen Gasgemisch-Zulauf- bzw. Ablaufleitungen 7, 13 jeweils hochdichte Klappen 15 auf; ebenso weisen die Zuluft- bzw. Abluftleitungen 3, 5 hochdichte Klappen 16 zur Steuerung der Raumlüftung auf. Somit kann Begasung bzw. Belüftung der einzelnen Räume 1 selektiv angesteuert werden.

In der teilweise aufgebrochenen Ansicht in Fig. 5 ist die Vorrichtung 2 mit dem zylindrischen Grundkörper 17 gezeigt, an dessen unterer Deckfläche 18 sechs Düsen 12 befestigt sind. Die Düsen 12 sind, wie insbesondere in Fig. 6 ersichtlich, entlang einer Kreisbahn im Wesentlichen gleich verteilt angeordnet, so dass die Achsen in horizontaler Richtung jeweils um 60° zueinander versetzt angeordnet sind. Bevorzugt kann der Durchmesser des Grundkörpers 17 ca. 270 mm betragen, jener Durchmesser einer von den Düsenöffnungen 11 definierten Einhüllenden ca. 390 mm.

Wie in Fig. 7 ersichtlich, kann in dem Grundkörper 17 der Hochdruckventilator 9 aufgenommen sein, über welchen über die Ansaugöffnungen 10 Umluft aus dem Raum 1 eingesaugt werden kann. Zugleich weist der Grundkörper 17, der an einer Reinraumdecke 19 befestigt ist, eine Anschlussöffnung auf, über welche somit hochkonzentriertes Wasserstoffperoxidgas in den Grundkörper 17 eingeleitet werden kann. Über die Leistungssteuerung des Hochdruckventilators 9 kann somit die gewünschte Konzentration an Reinigungsgas/Luft-Gemisch über die jeweilige Düse 12 unidirektional in den Raum 1 eingebracht werden. Um die Düsen 12 zeitlich versetzt zueinander, einzeln mit dem Reinigungsgas/LuftGemisch anzuspeisen, weisen diese jeweils ein Verschlussventil 21 (vgl. Fig. 5) auf. Anstelle der Befestigung an einer Reinraumdecke 19 kann der Grundkörper 17 samt der Düsen 12 auch beispielsweise auf einer höhenverstellbaren Hubsäule befestigt sein, so dass eine variable Begasungshöhe z.B. zwischen 1,7 und 3,3 m möglich ist. Sofern die Hubsäule mit einem mit feststellbaren Rädern versehenen Bodenelement verbunden ist, können die starr ausgerichteten Düsen 12 auf einfache Weise an verschiedenen Positionen in einem Raum angeordnet werden.

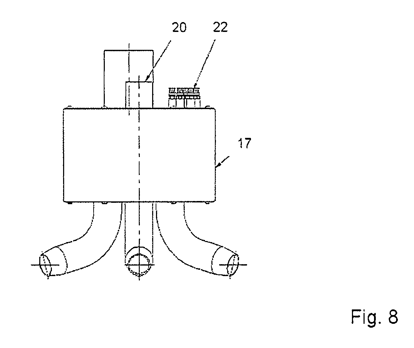

In Fig. 8 ist ein ähnliches Ausführungsbeispiel gezeigt, wobei hier die Höhe des Grundkörpers 17 niedriger ausgeführt ist, da kein Ventilator 9 aufgenommen ist. Hierbei wird an die Anschlussöffnung 20 eine Zulaufleitung 7 angeschlossen, über welche das bereits zuvor hergestellte Reinigungsgas/Luft-Gemisch direkt in den Raum 1 eingebracht wird. Zudem sind in Fig. 8 Anschlusszapfen 22 gezeigt, über welche Druckluftleitungen zur Steuerung der Verschlussventile 21 angeschlossen werden können.

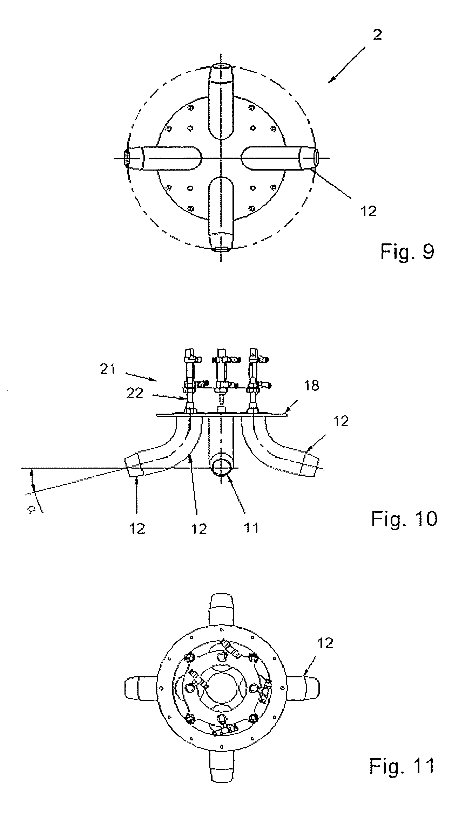

In den Fig. 9 bis 12 ist ein ähnliches Ausführungsbeispiel der Begasungsvorrichtung 2 gezeigt, wobei hier anstelle von sechs, lediglich vier entlang einer Kreisbahn gleichmäßig verteilt angeordnete Düsen 12 vorgesehen sind. Wie insbesondere in Fig. 10 ersichtlich, weisen die Düsen 12 anschließend an ihre Befestigung an die stirnseitige Platte 18 des Grundkörpers 17 einen im Längsschnitt kreisbogenförmigen Abschnitt 12' auf und endseitig einen sich konisch verjüngenden Endabschnitt 12''. Die Längsachse des sich konisch verjüngenden Endabschnittes 12'' ist hierbei in einem Winkel α von ca. 15° zur Horizontalen geneigt.

Den einzelnen Düsen 12 ist jeweils ein Verschlussventil 21 zugeordnet, welches jeweils einen Stößel 22 aufweist, der über eine pneumatische Steuer- bzw. Regelungsvorrichtung selektiv geöffnet und geschlossen werden kann. Somit kann jeweils über eine einzelne Düsenöffnung 11 unidirektional das Reinigungsgas/Luft-Gemisch in den Raum 1 eingebracht werden und somit die gewünschte großräumige stationäre Luftströmung in Form einer Walze erzielt werden.

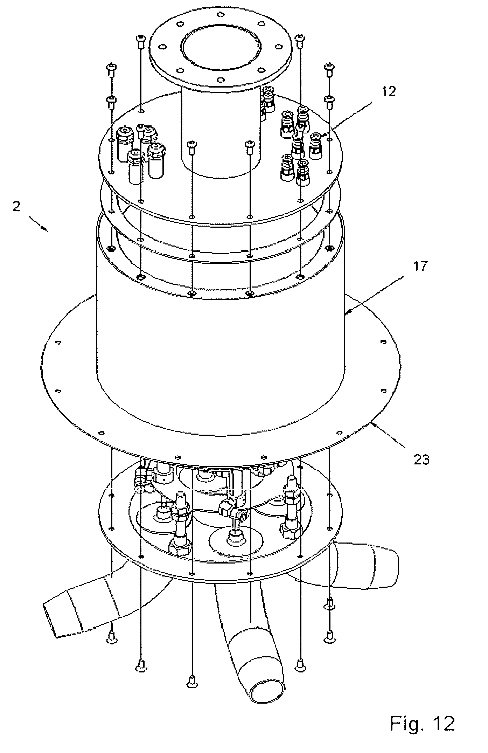

Wie insbesondere in Fig. 12 ersichtlich, ist mit dem Grundkörper 17 ein Befestigungsring 23 verbunden, über welchen auf einfache Weise eine Befestigung der gesamten Vorrichtung 2 an der Decke 19 des Raums 1 erzielt werden kann. Wesentlich ist jedoch lediglich, dass aufgrund der unidirektionalen Einbringung des Reinigungsgas/Luft-Gemisches über eine im Betrieb stationär angeordnete Düse 12 eine effiziente Dekontamination des Reinraums 1 erzielt werden kann. Die Vorrichtungen 2 können bei einem Rastermaß von ca. 60 cm bündig an der Reinraumdecke 19 befestigt werden, so dass eine Raumgröße von bis zu 100 m² bei einem ca. quaderförmigen Raum mit einer Höhe von 3 m effizient dekontaminiert werden kann. Nach Einbringung des Reinigungsgas/Luft-Gemisches kann in derselben Art und Weise, d.h. jeweils lediglich über eine einzige Düse 12, wobei die unterschiedlich ausgerichteten Düsen 12 intervallmäßig angesteuert werden, reine Luft in den Reinraum 1 eingebracht werden und somit eine Spülung des Reinraums 1 erzielt werden. Hierdurch kann gegenüber bekannten Spülvorgängen eine Zeitersparnis von bis zu 70% erzielt werden.

## Patentansprüche

1. Verfahren zur Dekontamination eines Raums (1) mit einem Reinigungsgas/Luft-Gemisch, **dadurch gekennzeichnet, dass** das Reinigungsgas/Luft-Gemisch über einen vorbestimmten Zeitraum im Wesentlichen unidirektional in den Raum (1) eingebracht wird, so dass sich ein Freistrahl ausbildet, wobei die unidirektionale Strömungsrichtung beibehalten wird, bis sich eine stationäre Strömung ausgebildet hat, und anschließend die unidirektionale Strömungsrichtung zur Reinigung des gesamten Raums (1) geändert wird.

2. Verfahren nach Anspruch 1, **dadurch gekennzeichnet, dass** nach einer vorbestimmten Einwirkzeit des Reinigungsgas/Luft-Gemisches reine Luft in den Raum (1) eingeleitet wird, wobei die Luft über einen vorbestimmten Zeitraum im Wesentlichen unidirektional in den Raum (1) eingeleitet wird, so dass sich ein Freistrahl ausbildet, wobei die unidirektionale Strömungsrichtung beibehalten wird, bis sich eine stationäre Strömung ausgebildet hat, und anschließend die unidirektionale Strömungsrichtung zur Spülung des gesamten Raums (1) geändert wird.

3. Verfahren nach Anspruch 1 oder 2, **dadurch gekennzeichnet, dass** die Konzentration des Reinigungsgase im Reinigungsgas/Luft-Gemisches vor Einbringung in den Raum (1) bestimmt wird.

4. Verfahren nach einem der Ansprüche 1 bis 3, **dadurch gekennzeichnet, dass** die unidirektionalen Strömungsrichtungen, die Gesamtdauer der Begasung sowie die Dauer der Intervalle der Begasung in jeweils eine bestimmte Strömungsrichtung, in Abhängigkeit von verschiedenen Parametern des Raums (1), insbesondere Größe, Raumform, und dergl., mittels Simulation per Computer vorbestimmt wird.

5. Vorrichtung (2) zur Dekontamination eines Raums (1) mit einem Reinigungsgas/Luft-Gemisch mit zumindest einer Düse (12), über welche das Reinigungsgas/Luft-Gemisch in den Raum (1) eingebracht wird, **dadurch gekennzeichnet, dass** die zumindest eine Düse (12) während der Einbringung des Reinigungsgas/Luft-Gemisches stationär angeordnet ist, so dass Reinigungsgas/Luft-Gemisch für einen vorbestimmten Zeitraum im Wesentlichen unidirektional über die Düse (12) in den Raum (1) einleitbar ist, wobei das Reinigungsgas/Luft-Gemisch über die zumindest eine Düse (12) zumindest in einer zweiten, unterschiedlichen Richtung unidirektional einleitbar ist.

6. Vorrichtung nach Anspruch 5, **dadurch gekennzeichnet, dass** zumindest zwei in unterschiedliche Richtungen ausgerichtete Düsen (12) vorgesehen sind, wobei jeder Düse (12) ein Verschlussventil (21) zugeordnet ist, so dass für einen vorbestimmten Zeitraum im Wesentlichen unidirektional über eine einzige Düse (12) das Reinigungsgas/Luft-Gemisch in den Raum (1) einleitbar ist.

7. Vorrichtung nach Anspruch 5 oder 6, **dadurch gekennzeichnet, dass** die Düsen (12) an einem, vorzugsweise zylindrischen, gemeinsamen Grundkörper (17) befestigt sind.

8. Vorrichtung nach Anspruch 7, **dadurch gekennzeichnet, dass** der Grundkörper (17) einen Anschluss (20) zur Einspeisung des Reinigungsgases und/oder des Reinigungsgas/Luftgemisches aufweist.

9. Vorrichtung nach Anspruch 7 oder 8, **dadurch gekennzeichnet, dass** die Düsen (12) entlang eine Kreisbahn in regelmäßigen Abständen angeordnet sind.

10. Vorrichtung nach einem der Ansprüche 7 bis 9, **dadurch gekennzeichnet, dass** die Ventile (21) jeweils einen in einer Schließstellung eine Eintrittsöffnung der jeweiligen Düse (12) verschließenden Stößel (22) aufweisen.

11. Vorrichtung nach Anspruch 10, **dadurch gekennzeichnet, dass** jedem Stößel (22) eine hydraulische, pneumatische oder elektrische Antriebsvorrichtung zugeordnet ist.

12. Vorrichtung nach Anspruch 11, **dadurch gekennzeichnet, dass** zur Ansteuerung der Antriebsvorrichtungen eine Steuer- bzw. Regeleinheit vorgesehen ist.

13. Vorrichtung nach einem der Ansprüche 7 bis 12, **dadurch gekennzeichnet, dass** in dem Grundkörper (17) ein Ventilator (9) aufgenommen ist.

14. Vorrichtung nach einem der Ansprüche 7 bis 13, **dadurch gekennzeichnet, dass** zur Mischung des Reinigungsgase und der Luft ein von dem Grundkörper getrennt angeordneter Behälter (14) vorgesehen ist.

15. Vorrichtung nach einem der Ansprüche 5 bis 14, **dadurch gekennzeichnet, dass** die gesamte Vorrichtung (2) aus Metall, inbesondere Edelstahl, oder einem Wasserstoffperoxid-beständigen Kunststoff, insbesondere PVC, PC, PTFE oder PE, besteht.
